# EUROPÄISCHE PATENTANMELDUNG

(11) **EP 1 933 132 A2**
(43) Veröffentlichungstag der Anmeldung: **18.06.2008**
(21) Anmeldenummer: 07122700.3
(22) Anmeldetag: 10.12.2007
(51) Int. Cl.: G01N 22/04, G01N 33/34

(54) **Verfahren und Vorrichtung zur Bestimmung der Feuchte einer laufenden Materialbahn**

(30) Priorität: 15.12.2006 DE 102006059308
(71) Anmelder: Voith Patent GmbH, 89522 Heidenheim (DE)
(72) Erfinder: Kaufmann, Oliver, Dr., 89522 Heidenheim (DE); Biener, Peter, 89542 Herbrechtingen (DE); Wenzl, Rainer, 73469 Riesburg (DE)

(57) **Zusammenfassung**

Bei einem Verfahren zur Bestimmung der Feuchte einer laufenden Materialbahn, insbesondere Papier- oder Kartonbahn, wird die Materialbahn mittels eines einen Mikrowellenresonator umfassenden Sensors abgetastet, das Resonanzverhalten des Mikrowellenresonators untersucht und anhand dieses Resonanzverhaltens unter Berücksichtigung des Abstandes zwischen dem Mikrowellenresonator und der Materialbahn die Feuchte der Materialbahn ermittelt. Es wird auch eine entsprechende Vorrichtung zur Bestimmung der Feuchte angegeben.

## Beschreibung

Die Erfindung betrifft ein Verfahren sowie eine Vorrichtung zur Bestimmung der Feuchte einer laufenden Materialbahn, insbesondere Papier- oder Kartonbahn.

Die Feuchte der laufenden Papierbahn ist eine der wichtigsten Mess- und Regelgrößen im Papierherstellungsprozess. Für schnelle Regelungen müssen die Messwerte online und präzise ermittelt werden. Dabei kann der gemessene Feuchtewert für Mess- und Regelaufgaben verwendet werden.

Derzeit wird die Feuchte in der Papierindustrie in der Regel mittels optischer Methoden bestimmt. Dabei werden charakteristische Wellenlängen von Wasser und Fasern spektroskopisch vermessen. Über das Transmissions- bzw. Reflexionsverhalten wird dann der prozentuale Feuchtegehalt des Papiers berechnet. Von Nachteil ist hierbei jedoch der hohe technische Aufwand, der hohe Preis und die großen Abmessungen der entsprechenden Sensoren.

Eine weitere in der Papierindustrie angewandte Technik zur Feuchtebestimmung beruht auf einem indirekten Verfahren wie der Messung der elektrischen Leitfähigkeit des Papiers, die in Korrelation zur im Papier enthaltenen Wassermenge steht (vgl. US 6,99,90). Die Nachteile dieser bekannten Methode bestehen darin, dass zum einen die Leitfähigkeit auch von der chemischen Zusammensetzung des Papiers abhängt und zum anderen die Messung von der dielektrischen Konstante des Papiers und vom Abstand des Sensorelements zum Papier abhängig ist.

Es wurde auch bereits vorgeschlagen, zur Feuchtemessung die Mikrowellen-Resonanzfrequenz eines in einem Sensor implementierten Hohlraumsensors zu bestimmen. Das Resonanzverhalten eines solchen Hohlraumresonators ändert sich je nach Feuchte des Papiers. Diese Methode weist unter anderem den Nachteil auf, dass der betreffende Sensor relativ große Abmessungen besitzt.

Mehrere Entwicklungen und Fortschritte in der Mikrowellentechnik für die Kommunikationsindustrie im für die Feuchtemessung wichtigen Frequenzbereich von 1 bis 100 GHz haben zu einer Miniaturisierung und zu einem Preisverfall der entsprechenden Komponenten geführt. Es eröffnen sich daher neue Wege für die weitere Entwicklung.

In der EP 1 000 314 B1 ist ein offener Hohlraumsensor zur Abstandsmessung beschrieben.

Der Erfindung liegt die Aufgabe zugrunde, ein verbessertes Verfahren sowie eine verbesserte Vorrichtung der eingangs genannten Art zu schaffen, die auf möglichst einfache und kostengünstige Art und Weise eine zuverlässige Bestimmung der Feuchte bzw. des Wassergewichts der laufenden Materialbahn ermöglichen. Dabei soll insbesondere eine im Vergleich zu den bisher üblichen kostspieligen optischen Verfahren und Vorrichtungen kostengünstigere und zuverlässigere Alternative geschaffen werden.

Erfindungsgemäß wird diese Aufgabe gelöst durch ein Verfahren zur Bestimmung der Feuchte einer laufenden Materialbahn, insbesondere Papier- oder Kartonbahn, bei dem die Materialbahn mittels eines einen Mikrowellenresonator umfassenden Sensors abgetastet, das Resonanzverhalten des Mikrowellenresonators untersucht und anhand dieses Resonanzverhaltens unter Berücksichtigung des Abstandes zwischen dem Mikrowellenresonator und der Materialbahn die Feuchte der Materialbahn ermittelt wird.

Damit kann nunmehr das Wassergewicht einer laufenden Materialbahn, insbesondere Papier- oder Kartonbahn, unabhängig vom Abstand des Sensors zur Materialbahn zuverlässig bestimmt werden. Mit dem erfindungsgemäßen Verfahren wird eine kostengünstige und zuverlässige Alternative zu den bisher üblichen kostspieligen optischen Verfahren bereitgestellt. Der hierbei verwendete Mikrowellenresonator zeichnet sich u.a. durch geringe Kosten, kleine Abmessungen und dadurch aus, dass keine Temperaturabhängigkeit durch den Sensor gegeben ist.

Es kann also das für einen Mikrowellenresonator typische Messprinzip der Ausmessung der Resonanzfrequenz bestimmter ausgewählter Moden auch für die Feuchtemessung herangezogen werden. Indem der Abstand des Sensors zur Materialbahn beispielsweise konstant gehalten oder beispielsweise mittels eines zusätzlichen Sensors gleichzeitig mit ermittelt wird und dieser Abstand demzufolge genau bekannt ist, kann die Abstandsabhängigkeit der durch den Mikrowellenresonator durchgeführten Messung problemlos kompensiert werden.

Das erfindungsgemäße Verfahren kann beispielsweise zur Papierfeuchtemessung, zur Regelung verschiedener Querprofile oder zur Optimierung der Entwässerung in Papierlaufrichtung eingesetzt werden. Es ist beispielsweise auch ein Einsatz des Verfahrens zur Bändelmessung für eine Optimierung der Papierüberführung durch eine entsprechende Regelung denkbar, wobei der Feuchtewert als Istwert in den betreffenden Regelkreis eingeht. Es ist beispielsweise auch ein Einsatz des erfindungsgemäßen Verfahrens zur so genannten Gelpunktmessung beispielsweise für eine Optimierung der Trocknung eines durch Streichen veredelten Papiers durch eine entsprechende Regelung der Trockenleistungen möglich. Dabei geht der Feuchtewert als Istwert in den betreffenden Regelkreis ein. Die Feuchte korreliert mit dem so genannten Gelpunkt des Strichs, der die Fixierung oder Immobilisierung des Strichs widerspiegelt.

Bevorzugt wird als Mikrowellenresonator ein Resonator in Form eines Hohlraumresonators mit Resonatorgehäuse eingesetzt. Dabei kann der Hohlraumresonator insbesondere so beschaffen sein, wie dies in der EP 1 000 314 B1 beschrieben ist.

Der Hohlraumresonator kann also beispielsweise eine koplanare Schlitzkopplung mit Einkopplungsleitung aufweisen, die an dem Resonatorgehäuse abgeschlossen ist. Alternativ kann der Hohlraumresonator beispielsweise auch eine Mikrostreifenleitung zur Einkopplung aufweisen, die an dem Resonatorgehäuse abgeschlossen ist. Auch im übrigen kann der Hohlraumresonator vorteilhafterweise so ausgelegt sein, wie dies in der EP 1 000 314 B1 beschrieben ist.

Von Vorteil ist insbesondere auch, wenn die Resonanzfrequenz des Mikrowellenresonators für einen Abstand zwischen dem Mikrowellenresonator und der Materialbahn, wie er während der Abtastung der Materialbahn gegeben ist, in einem Bereich zwischen etwa 1 und 100 GHz liegt. Es kann also beispielsweise zur Feuchtemessung an einer laufenden Papierbahn das Resonanzverhalten des Mikrowellenresonators in einem Wellenlängenbereich von 1 GHz bis 100 GHz untersucht werden.

Die Untersuchung des Resonanzverhaltens des Mikrowellenresonators umfasst bevorzugt die Feststellung der Lage des Minimums/Maximums, d.h. eines Extremwerts der Resonanzkurve auf der Frequenzachse und/oder die Feststellung der Breite der Resonanzkurve. Damit wird dem Umstand Rechnung getragen, dass die Lage des Minimums/Maximums, d.h. eines Extremwerts der Resonanzkurve auf der Frequenzachse sowie die Breite dieser Resonanzkurve bzw. die Dämpfung vom Feuchtegehalt beispielsweise des Papiers abhängt.

Zur Sicherstellung einer optimalen Funktion des Sensors ist die den zur Materialbahn hin offenen Mikrowellenresonator und die Materialbahn umfassende Einheit abgeschlossen. Vorteilhafterweise kann dazu in einem festen Abstand von der der Materialbahn zugewandten offenen bzw. nicht metallisierten oder nicht dielektrischen Seite des Mikrowellenresonators ein metallisches oder dielektrisches Element positioniert werden.

Gemäß einer zweckmäßigen praktischen Ausgestaltung des erfindungsgemäßen Verfahrens wird der Abstand zwischen dem Mikrowellenresonator und der Materialbahn zumindest im Wesentlichen konstant gehalten. In diesem Fall kann dann dieser bekannte konstante Abstand zur Kompensation der Abstandsabhängigkeit der Sensormessung herangezogen werden.

Insbesondere in dem Fall, dass eine einseitige Messung der Materialbahn vorgesehen ist, wird gemäß einer zweckmäßigen praktischen Ausgestaltung nur ein einen Mikrowellenresonator umfassender Sensor eingesetzt und der Abstand zwischen diesem Sensor und der Materialbahn zumindest im Wesentlichen konstant gehalten.

Die maximal zulässige Abweichung des Abstandes vom Sollabstand ist vorzugsweise < 100 pm, vorzugsweise < 10 µm.

Gemäß einer weiteren zweckmäßigen Ausgestaltung können vorteilhafterweise auch zwei auf derselben Seite der Materialbahn vorgesehene jeweils einen Mikrowellenresonator umfassende Sensoren eingesetzt werden, die bezüglich der Feuchte und des Abstandes unterschiedliche Empfindlichkeiten besitzen, wobei mittels eines der beiden Sensoren die Feuchte und mittels des anderen Sensors gleichzeitig der Abstand zwischen dem Mikrowellenresonator und der Materialbahn ermittelt wird. Die unterschiedlichen Empfindlichkeiten können beispielsweise über unterschiedliche Messfrequenzen gegeben sein.

Bevorzugt werden die beiden Sensoren unmittelbar nebeneinander positioniert.

Der Abstand zwischen einem jeweiligen Sensor und der Materialbahn beträgt zweckmäßigerweise maximal 200 mm, wobei er vorzugsweise in einem Bereich von etwa 1 bis etwa 10 mm liegt.

Unabhängig davon, ob lediglich ein oder mehrere jeweils einen Mikrowellenresonator umfassende Sensoren eingesetzt werden, wird vorteilhafterweise gleichzeitig die Temperatur gemessen. Damit kann insbesondere dem Umstand Rechnung getragen werden, dass die dielektrische Konstante von Wasser sich mit der Temperatur ändert.

Bevorzugt wird die Temperatur der Materialbahn berührungslos gemessen.

Die den Mikrowellenresonator und die Materialbahn umfassende Einheit wird zweckmäßigerweise durch eine insbesondere metallische Stützfläche wie beispielsweise den metallischen Kern einer Walze abgeschlossen, über die die Materialbahn geführt ist. Dabei kann zwischen der Materialbahn und der Stützfläche eine Bespannung, insbesondere ein Sieb vorgesehen sein.

Vorteilhafterweise wird für eine Abgleichsmessung ohne Materialbahn wenigstens ein zusätzlicher einen Mikrowellenresonator umfassender Sensor eingesetzt, um insbesondere eine Stützfläche bzw. Walze oder Bespannung an einer nicht mehr von der Materialbahn berührten Stelle abzutasten, wodurch die Messgenauigkeit entsprechend erhöht wird. Es können so beispielsweise Verschmutzungen der Stützfläche bzw. Walze oder der beispielsweise durch ein Sieb gebildeten Bespannung kompensiert werden.

Von Vorteil ist hierbei, wenn der zusätzliche Sensor quer zur Bahnlaufrichtung betracht zumindest im Wesentlichen mit einem die Materialbahn abtastenden Sensor ausgerichtet ist.

Soll eine zweiseitige Messung durchgeführt werden, so wird vorteilhafterweise an einem freien Zug der Materialbahn, an dem diese nicht abgestützt ist, auf jeder den beiden einander gegenüberliegenden Seiten der Materialbahn jeweils wenigstens ein einen Mikrowellenresonator umfassender Sensor eingesetzt.

Hierbei ist es von Vorteil, wenn die auf entgegengesetzten Seiten der Materialbahn vorgesehenen Sensoren in Bahnlaufrichtung betrachtet zumindest soweit gegeneinander versetzt sind, dass sich deren elektromagnetische Felder nicht gegenseitig beeinflussen. Die beiden Sensoren sollen also möglichst nicht direkt übereinander angeordnet werden.

Auch in diesem Fall ist eine jeweilige einen zur Materialbahn hin offenen Mikrowellenresonator und die Materialbahn umfassende Einheit zweckmäßigerweise wieder abgeschlossen. Dazu kann beispielsweise jeweils wieder in einem festen Abstand von der der Materialbahn zugewandten offenen bzw. nicht metallisierten oder nicht dielektrischen Seite des jeweiligen Mikrowellenresonators ein metallisches oder dielektrisches Element positioniert werden.

Bevorzugt wird eine Anpassung der Sensorkennlinie und/oder Kalibration der Sensorwerte bezüglich der absoluten Feuchte vorgenommen.

Zur Ermittlung des in Prozent Feuchte von der Gesamtmasse angegebenen Feuchtegehalts kann zusätzlich das Flächengewicht der Materialbahn gemessen werden. In der Papierindustrie ist dieser Feuchtegehalt die übliche betreffende Größe.

Hierbei ist es von Vorteil, wenn die Feuchtemessung in Bahnlaufrichtung betrachtet in einem Abstand von dem zur Bestimmung der Feuchte eingesetzten Sensor erfolgt, in dem sich die Feuchte der Materialbahn gegenüber der Feuchte der Materialbahn im Bereich des zur Feuchtebestimmung eingesetzten Sensors im Wesentlichen nicht ändert oder die Feuchteänderung bekannt ist.

Erfindungsgemäß wird die weiter oben angegebene Aufgabe überdies gelöst durch eine Vorrichtung zur Bestimmung der Feuchte einer laufenden Materialbahn, insbesondere Papier- oder Kartonbahn, mit einem einen Mikrowellenresonator umfassenden Sensor zur Abtastung der Materialbahn und einer Auswerteelektronik zur Untersuchung des Resonanzverhaltens des Mikrowellenresonators sowie zur Ermittlung der Feuchte der Materialbahn anhand des Resonanzverhaltens unter Berücksichtigung des Abstandes zwischen dem Mikrowellenresonator und der Materialbahn.

Bevorzugte Ausführungsformen der erfindungsgemäßen Vorrichtung sind in den Unteransprüchen angegeben.

Die erfindungsgemäße Vorrichtung ist insbesondere zur Durchführung des erfindungsgemäßen Verfahrens vorgesehen.

Das erfindungsgemäße Verfahren und die erfindungsgemäße Vorrichtung sind vorteilhafterweise insbesondere zur Ermittlung der Feuchte eines Papierrandstreifens zur Optimierung der Überführung einer Papierbahn von einem Bereich einer Papiermaschine auf einem anderen verwendbar.

Eine bevorzugte vorteile Anwendung besteht also im Bereich der so genannten Bändelmessung, wobei eine Feuchtemessung eines Papierrandstreifens zur Überführungsoptimierung des Papiers von einem Bereich der Papiermaschine auf einen anderen erfolgt. Dabei kann die Feuchtemessung als Istwert in einem Regelkreis dienen, mit dem die Papierfeuchte auf einen Ideal- oder Sollwert für die Überführung eingestellt werden kann. Als Stellgrößen können entweder Papierrückbefeuchtungsaktuatoren für eine Steigerung der Feuchte oder die Kontrolle der Dampfmenge in den Trockenpartien sowohl für eine Steigerung der Feuchte als auch für die Papiertrocknung verwendet werden.

Das erfindungsgemäße Verfahren sowie die erfindungsgemäße Vorrichtung sind vorteilhafterweise insbesondere auch bei der Veredelung von Papier, wie insbesondere dem Streichen von Papier, und dabei insbesondere zur Gelpunktbestimmung verwendbar.

Eine beispielhafte bevorzugte Anwendung liegt demzufolge im Bereich der Gelpunktmessung. So spielt bei der Veredelung des Papiers, wie z.B. dem Streichen des Papiers, die Papierfeuchte eine bedeutende Rolle. Es besteht eine Korrelation zwischen dem so genannten Gelpunkt des auf das Papier aufgetragenen Strichs und der Feuchte. Dabei bestimmt der Gelpunkt, wann der Strich fixiert ist und entsprechend der Streichvorgang sein Ende findet, was für die nachfolgenden Trockengruppen von entscheidender Bedeutung ist.

Das erfindungsgemäße Verfahren sowie die erfindungsgemäße Vorrichtung sind mit Vorteil insbesondere auch zur Quer- und/oder Längsfeuchteprofilregelung verwendbar.

Eine bevorzugte Anwendung liegt also beispielsweise auch allgemein im Bereich der Feuchtemessung und der damit einhergehenden Quer- und Längsprofilregelung. Es können verschiedene Querprofilregelungen mit den erhaltenen Sensormesswerten durchgeführt werden. Ein entscheidender Vorteil des eingesetzten Mikrowellenresonators besteht in dessen geringer Größe und den geringen Herstellungskosten, wodurch es möglich ist, eine die gesamte Papierbahnbreite erfassende Sensorleiste herzustellen. Die Vorteile gegenüber herkömmlichen traversierenden Sensoreinheiten bestehen zum einen darin, dass die Feuchte der gesamten Papierbahnbreite auf einmal ermittelt werden kann, und zum andern darin, dass 100 % der Papierbahn vermessen werden können. Dagegen wird bei den herkömmlichen traversierenden Messeinheiten stets nur ein Ausschnitt bzw. ein diagonaler Streifen über die Papierbahnbreite gemessen. Grundsätzlich kann der erfindungsgemäß eingesetzte Sensor, beispielsweise aus Kosten- oder Kompatibilitätsgründen, aber auch in eine traversierende Messeinrichtung eingebaut werden.

Die Erfindung wird im Folgenden anhand von Ausführungsbeispielen unter Bezugnahme auf die Zeichnung näher erläutert; in dieser zeigen:
- Fig.1 1: eine schematische Darstellung einer beispielhaften Ausführungsform einer Vorrichtung zur Bestimmung der Feuchte einer laufenden Materialbahn für eine einseitige Messung mit zwei auf einer Seite der Materialbahn vorgesehenen Sensoren zur Feuchte- bzw. Abstandsmessung,
- Fig. 2: eine mit der Ausführungsform gemäß Fig. 1 vergleichbare Ausführungsform mit einem zusätzlichen Sensor für eine Abgleichsmessung ohne Materialbahn,
- Fig. 3: eine schematische Darstellung einer weiteren Ausführungsform der Vorrichtung für eine zweiseitige Messung, bei der an einem freien Zug der Materialbahn auf jeder der beiden einander gegenüberliegenden Seiten der Materialbahn jeweils wenigstens ein Sensor vorgesehen ist,
- Fig.4: eine mit der Ausführungsform gemäß Fig. 3 vergleichbare Ausführungsform der Vorrichtung, wobei im vorliegenden Fall jedoch eine die beiden Sensoren umfassende traversierende Einheit vorgesehen ist,
- Fig. 5: eine schematische Darstellung einer weiteren Ausführungsform der Vorrichtung mit nur einem in einem festen Abstand zur Materialbahn angeordneten Sensor an einem freien Zug der Materialbahn, wobei die den Sensor bzw. den zugeordneten Mikrowellenresonator und die Materialbahn umfassende Einheit durch ein metallisches oder dielektrisches Element abgeschlossen ist,
- Fig. 6: eine mit der Ausführungsform gemäß Fig. 5 vergleichbare Ausführungsform der Vorrichtung, wobei im vorliegenden Fall jedoch eine den Sensor und das metallische oder dielektrische Element umfassende traversierende Einheit vorgesehen ist,
- Fig. 7: eine schematische Darstellung einer weiteren Ausführungsform der Vorrichtung, die für eine Bändelmessung verwendet wird und
- Fig. 8: eine schematische Darstellung eines Regelkreises für eine Optimierung der Überführung einer Papierbahn auf der Basis einer Bändelmessung.

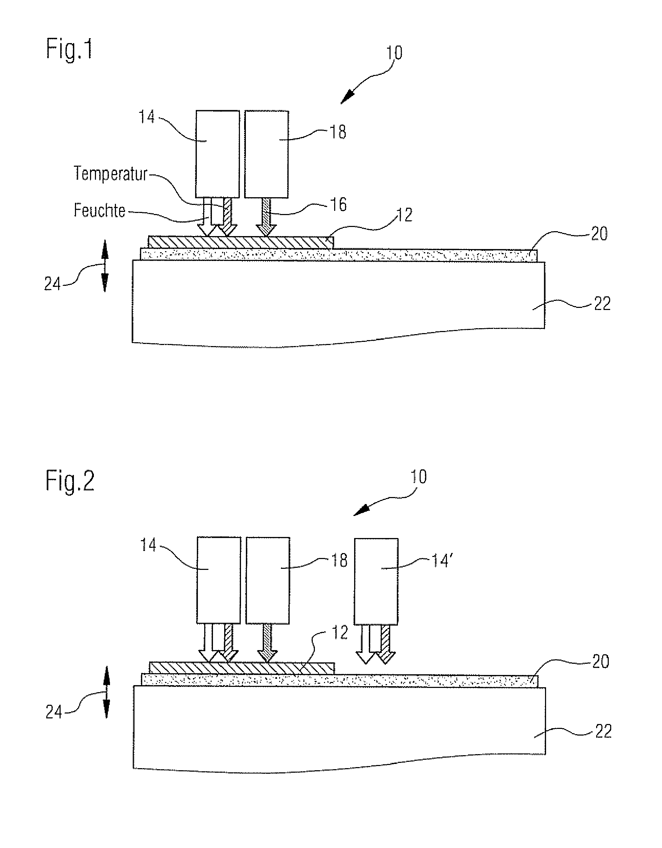

Fig. 1 zeigt in schematischer Darstellung eine beispielhafte Ausführungsform einer Vorrichtung 10 zur Bestimmung der Feuchte einer laufenden Materialbahn, bei der es sich im vorliegenden Fall beispielsweise um eine Papierbahn 12 handelt.

Die Vorrichtung 10 umfasst einen einen Mikrowellenresonator enthaltenden Sensor zur Messung der Feuchte der Papierbahn 12 sowie eine Auswerteelektronik (nicht gezeigt) zur Untersuchung des Resonanzverhaltens des Mikrowellenresonators sowie zur Ermittlung der Feuchte der Papierbahn 12 anhand des Resonanzverhaltens unter Berücksichtigung des Abstandes 16 zwischen dem Mikrowellenresonator und der Materialbahn 12. Der Sensor 14 umfasst überdies Mittel für eine gleichzeitige, vorzugsweise berührungslose Messung der Temperatur der Papierbahn 12.

Auf derselben Seite der Papierbahn 12 ist ein zweiter einen Mikrowellenresonator umfassender Sensor 18 zur Messung des Abstandes zur Papierbahn 12 vorgesehen. Dabei kann der Sensor 18 bzw. dessen Mikrowellenresonator einen gleichen Abstand 16 zur Papierbahn 12 wie der Sensor 14 bzw. der diesem zugeordnete Mikrowellenresonator besitzen, so dass mit dem über dem Sensor 18 erhaltenen Abstandsmesswert auch der Abstand des Sensors 14 bzw. dessen Mikrowellenresonator zur Papierbahn 12 erfasst wird.

Die beiden auf derselben Seite der Papierbahn 12 vorgesehenen jeweils einen Mikrowellenresonator umfassenden Sensoren 14, 18 können bezüglich der Feuchte und des Abstandes unterschiedliche Empfindlichkeiten, z.B. über unterschiedliche Messfrequenzen, besitzen. Dabei wird, wie bereits erwähnt, mittels des zweiten Sensors 18 gleichzeitig der Abstand zur Papierbahn 12 gemessen.

Im vorliegenden Fall erfolgt eine einseitige Messung. Auf der den beiden Sensoren 14, 18 gegenüberliegenden Seite ist die Papierbahn 12 auf einer Bespannung, hier beispielsweise einem Sieb 20 abgestützt, das über eine mit der betreffenden Bespannung versehene Walze 22 geführt ist.

Die Mikrowellenresonatoren der Sensoren 14, 18 sind jeweils zur Papierbahn 12 hin offen. Sie besitzen also jeweils der Papierbahn 12 zugewandt eine nicht metallisierte und nicht dielektrische Seite. Im vorliegenden Fall sind die durch einen jeweiligen Sensor 14 bzw. 18 bzw. einen jeweiligen Mikrowellenresonator und die Papierbahn 12 gebildeten Einheiten durch den metallischen Kern der Walze 22 abgeschlossen.

Nachdem der Abstand zwischen einem jeweiligen Mikrowellenresonator und der Papierbahn 12 bekannt ist, kann insbesondere über die Auswerteelektronik die Abstandsabhängigkeit der Mikrowellenresonatoren kompensiert werden. Eine jeweilige Abstandsänderung 24 wird über den Sensor 18 erfasst. Die maximalen Abstandsänderungen sind zweckmäßigerweise < 100 µm und vorzugsweise < 10 µm.

Die beiden Sensoren 14, 18 sind quer zur Bahnlaufrichtung betrachtet direkt nebeneinander positioniert. Der Abstand zu der zu untersuchenden Papierbahn 12 kann dabei bis zu 200 mm betragen, wobei er bevorzugt in einem Bereich von etwa 1 bis etwa 10 mm liegt.

Indem über den Sensor 14 gleichzeitig auch die Temperatur der Papierbahn 12 gemessen wird, kann insbesondere auch dem Umstand Rechnung getragen werden, dass sich die dielektrische Konstante von Wasser mit der Temperatur ändert.

Fig. 2 zeigt eine mit der Ausführungsform gemäß Fig. 1 vergleichbare Ausführungsform mit einem zusätzlichen, einen Mikrowellenresonator umfassenden Sensor 14' für eine Abgleichsmessung ohne Papierbahn 12. Dadurch kann beispielsweise die Verschmutzung des Siebs 20 gemessen und insbesondere mittels der Auswerteelektronik entsprechend kompensiert werden. Dabei sollte der zusätzliche Sensor 14' quer zur Bahnlaufrichtung betrachtet zumindest im Wesentlichen mit dem zur Messung der Feuchte vorgesehenen Sensor 14 ausgerichtet sein.

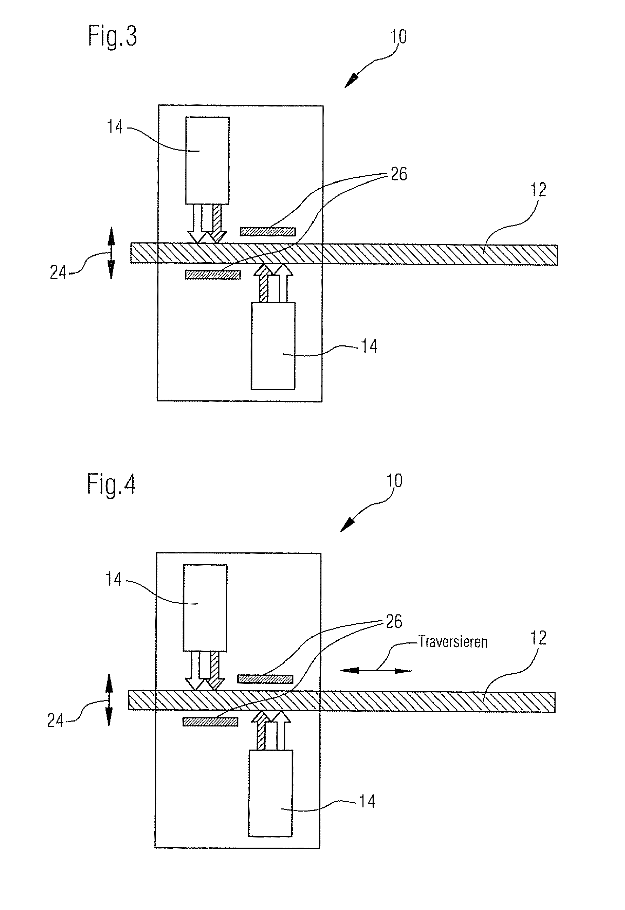

Fig. 3 zeigt in schematischer Darstellung eine weitere Ausführungsform der Vorrichtung 10 für eine zweiseitige Messung, bei der an einem freien Zug der Papierbahn 12 auf jeder der beiden einander gegenüberliegenden Seiten der Papierbahn 12 jeweils ein einen Mikrowellenresonator zur Messung der Feuchte umfassender Sensor 14 vorgesehen ist. Mittels der Sensoren 14 wird gleichzeitig auch jeweils wieder die Temperatur der Papierbahn 12 gemessen. Jede der einen jeweiligen Sensor 14 bzw. einen jeweiligen Mikrowellenresonator und die Papierbahn 12 umfassenden Einheiten ist hier durch ein metallisches oder dielektrisches Element abgeschlossen, das im vorliegenden Fall jeweils durch eine entsprechende Abschlussplatte 26 gebildet ist, die hier beispielsweise aus Metall besteht.

Fig. 4 zeigt eine mit der Ausführungsform gemäß Fig. 3 vergleichbare Ausführungsform der Vorrichtung 10, wobei im vorliegenden Fall jedoch eine die beiden Sensoren 14 und die Abschlussplatten 26 umfassende traversierende Einheit vorgesehen ist.

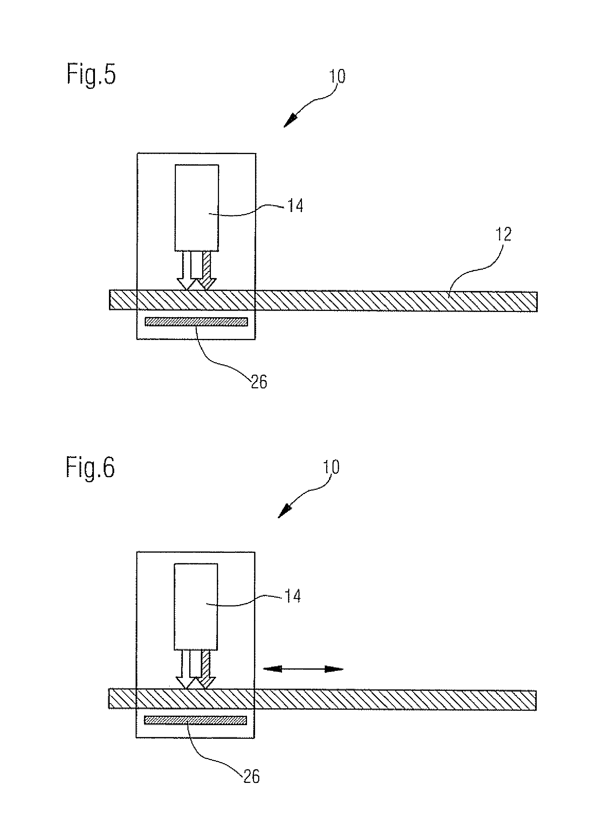

Fig. 5 zeigt in schematischer Darstellung eine weitere Ausführungsform der Vorrichtung 10 mit nur einem in einem festen Abstand zur Papierbahn 12 angeordneten, einen Mikrowellenresonator umfassenden Sensor 14 an einem freien Zug der Materialbahn 12. Die den Sensor 14 bzw. den zugeordneten Mikrowellenresonator und die Papierbahn 12 umfassende Einheit ist auch im vorliegenden Fall wieder durch ein metallisches oder dielektrisches Element, hier beispielsweise eine vorzugsweise aus Metall bestehende Abschlussplatte 26 abgeschlossen.

Fig. 6 zeigt eine mit der Ausführungsform gemäß Fig. 5 vergleichbare Ausführungsform der Vorrichtung 10, wobei im vorliegenden Fall jedoch eine den Sensor 14 bzw. deren Mikrowellenresonator und die Abschlussplatte 26 umfassende traversierende Einheit vorgesehen ist.

Wie zuvor dienen die Sensoren 14 der in den Fig. 5 und 6 wiedergegebenen Ausführungsformen jeweils wieder sowohl der Messung der Feuchte als auch der Messung der Temperatur.

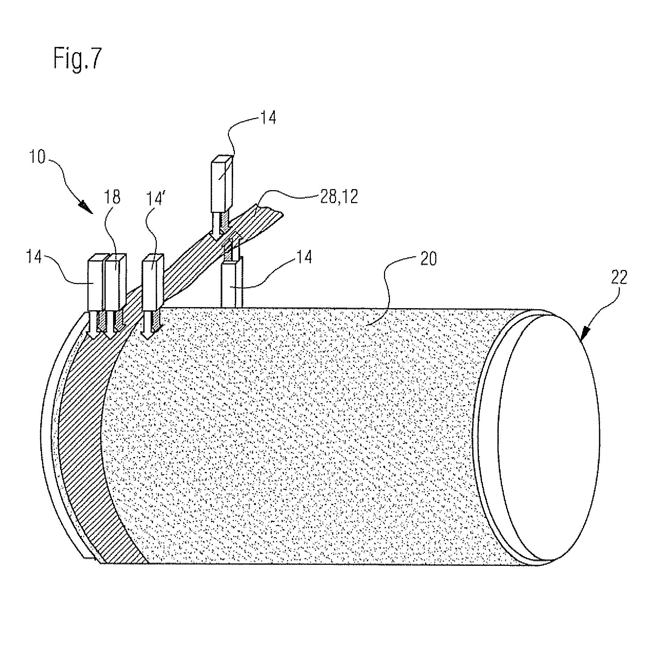

Fig. 7 zeigt in schematischer Darstellung eine weitere Ausführungsform der Vorrichtung 10, die für eine Bändelmessung verwendet wird.

Wie anhand der Fig. 7 zu erkennen ist, sind dem Bändel oder Papierrandstreifen 28 der Papierbahn 12 in dem Bereich, in dem er über eine mit einer Bespannung 20 versehene Walze 22 geführt wird, für eine zunächst einseitige Messung wieder zwei auf der von der Walze 22 abgewandten Seite vorgesehene, jeweils einen Mikrowellenresonator umfassende Sensoren 14, 18 zur Feuchte-, Temperatur-und Abstandsmessung zugeordnet. Zudem ist ein zusätzlicher Sensor 14' für eine Abgleichsmessung ohne Papierbahn 12 vorgesehen. Für diese einseitige Messung ist also wieder eine mit der Sensoranordnung gemäß Fig. 2 vergleichbare Sensoranordnung vorgesehen.

Zudem ist an einem freien Zug des Papierrandstreifens 28 der Papierbahn 12 für eine zweiseitige Messung auf jeder der beiden einander gegenüberliegenden Seiten des Papierrandstreifens 28 jeweils ein weiterer einen Mikrowellenresonator umfassender Sensor 14 vorgesehen. Für diese zweiseitige Messung ist also eine mit der Sensoranordnung gemäß Fig. 3 vergleichbare Sensoranordnung vorgesehen.

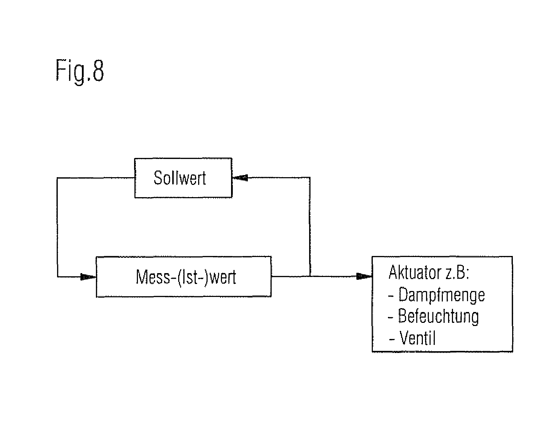

Fig. 8 zeigt in schematischer Darstellung einen beispielhaften Regelkreis für eine Optimierung der Überführung einer Papierbahn auf der Basis einer Bändelmessung.

In diesem Regelkreis dient die Feuchtemessung als Istwert. Die Papierfeuchte wird durch den Regelkreis auf einen Ideal- oder Sollwert für die Überführung eingestellt. Als Stellgrößen können entweder Papierrückbefeuchtungsaktuatoren für eine Steigerung der Feuchte und/oder die Kontrolle der Dampfmenge in den Trockenpartien sowohl für eine Steigerung der Feuchte als auch für die Papiertrocknung verwendet werden.

### Bezugszeichenliste

- 10: Vorrichtung zur Bestimmung der Feuchte
- 12: Materialbahn, Papier- oder Kartonbahn
- 14: Sensor
- 14': Sensor
- 16: Abstand
- 18: Sensor
- 20: Bespannung, Sieb
- 22: Walze
- 24: Abstandsänderung
- 26: metallisches oder dielektrisches Element, Abschlussplatte
- 28: Bändel, Papierrandstreifen

## Patentansprüche

1. Verfahren zur Bestimmung der Feuchte einer laufenden Materialbahn (12), insbesondere Papier- oder Kartonbahn, bei dem die Materialbahn (12) mittels eines einen Mikrowellenresonator umfassenden Sensors (14) abgetastet, das Resonanzverhalten des Mikrowellenresonators untersucht und anhand dieses Resonanzverhaltens unter Berücksichtigung des Abstandes (16) zwischen dem Mikrowellenresonator und der Materialbahn (12) die Feuchte der Materialbahn (12) ermittelt wird.

2. Verfahren nach Anspruch 1,
**dadurch gekennzeichnet ,**
**dass** als Mikrowellenresonator ein Resonator in Form eines Hohlraumresonators mit Resonatorgehäuse eingesetzt wird.

3. Verfahren nach Anspruch 1 oder 2,
**dadurch gekennzeichnet,**
**dass** die Resonanzfrequenz des Mikrowellenresonators für einen Abstand (16) zwischen dem Mikrowellenresonator und der Materialbahn (12), wie er während der Abtastung der Materialbahn (12) gegeben ist, in einem Bereich zwischen etwa 1 und 100 GHz liegt.

4. Verfahren nach einem der vorhergehenden Ansprüche,
**dadurch gekennzeichnet,**
**dass** die Untersuchung des Resonanzverhaltens des Mikrowellenresonators die Feststellung der Lage des Minimums/Maximums der Resonanzkurve auf der Frequenzachse und/oder die Feststellung der Breite der Resonanzkurve umfasst.

5. Verfahren nach einem der vorhergehenden Ansprüche,
**dadurch gekennzeichnet ,**
**dass** die den zur Materialbahn (12) hin offenen Mikrowellenresonator und die Materialbahn (12) umfassende Einheit abgeschlossen ist.

6. Verfahren nach Anspruch 5,
**dadurch gekennzeichnet,**
**dass** in einem festen Abstand (16) von der der Materialbahn (12) zugewandten offenen bzw. nicht metallisierten oder nicht dielektrischen Seite des Mikrowellenresonators ein metallisches oder dielektrisches Element (22, 26) positioniert wird.

7. Verfahren nach einem der vorhergehenden Ansprüche,
**dadurch gekennzeichnet,**
**dass** der Abstand (16) zwischen dem Mikrowellenresonator und der Materialbahn (12) zumindest im Wesentlichen konstant gehalten wird.

8. Verfahren nach einem der vorhergehenden Ansprüche,
**dadurch gekennzeichnet,**
**dass** nur ein einen Mikrowellenresonator umfassender Sensor (14) eingesetzt und der Abstand (16) zwischen diesem Sensor und der Materialbahn zumindest im Wesentlichen konstant gehalten wird.

9. Verfahren nach Anspruch 8,
**dadurch gekennzeichnet,**
**dass** die maximal zulässige Abweichung des Abstandes (16) vom Sollabstand < 100m, vorzugsweise < 10 mmist.

10. Verfahren nach einem der Ansprüche 1 bis 7,
**dadurch gekennzeichnet,**
**dass** zwei auf derselben Seite der Materialbahn vorgesehene jeweils einen Mikrowellenresonator umfassende Sensoren (14, 18) eingesetzt werden, die bezüglich der Feuchte und des Abstandes unterschiedliche Empfindlichkeiten besitzen, und dass mittels eines (14) der beiden Sensoren (14, 18) die Feuchte und mittels des anderen Sensors (18) gleichzeitig der Abstand (16) zwischen dem Mikrowellenresonator und der Materialbahn (12) ermittelt wird.

11. Verfahren nach Anspruch 10,
**dadurch gekennzeichnet,**
**dass** die beiden Sensoren (14, 18) unmittelbar nebeneinander positioniert werden.

12. Verfahren nach Anspruch 10 oder 11,
**dadurch gekennzeichnet,**
**dass** der Abstand (16) zwischen einem jeweiligen Sensor (14, 18) und der Materialbahn (12) maximal 200 mm beträgt und vorzugsweise in einem Bereich von etwa 1 bis etwa 10 mm liegt.

13. Verfahren nach einem der vorhergehenden Ansprüche,
**dadurch gekennzeichnet ,**
**dass** gleichzeitig die Temperatur der Materialbahn (12) gemessen wird.

14. Verfahren nach Anspruch 13,
**dadurch gekennzeichnet,**
**dass** die Temperatur der Materialbahn (12) berührungslos gemessen wird.

15. Verfahren nach einem der vorhergehenden Ansprüche,
**dadurch gekennzeichnet ,**
**dass** die den Mikrowellenresonator und die Materialbahn (12) umfassende Einheit durch eine insbesondere metallische Stützfläche wie insbesondere den metallischen Kern einer Walze (22) abgeschlossen ist, über die die Materialbahn (12) geführt ist.

16. Verfahren nach Anspruch 15,
**dadurch gekennzeichnet,**
**dass** zwischen der Materialbahn (12) und der Stützfläche bzw. Walze (22) eine Bespannung (20), insbesondere ein Sieb vorgesehen ist.

17. Verfahren nach einem der vorhergehenden Ansprüche,
**dadurch gekennzeichnet,**
**dass** für eine Abgleichsmessung ohne Materialbahn (12) wenigstens ein zusätzlicher einen Mikrowellenresonator umfassender Sensor (14') eingesetzt wird, um insbesondere eine Stützfläche bzw. Walze (22) oder Bespannung (20) an einer nicht von der Materialbahn (12) berührten Stelle abzutasten.

18. Verfahren nach Anspruch 17,
**dadurch gekennzeichnet,**
**dass** der zusätzliche Sensor (14') quer zur Bahnlaufrichtung betrachtet zumindest im Wesentlichen mit einem die Materialbahn (12) abtastenden Sensor (14, 18) ausgerichtet ist.

19. Verfahren nach einem der vorhergehenden Ansprüche,
**dadurch gekennzeichnet,**
**dass** an einem freien Zug der Materialbahn (12) auf jeder den beiden einander gegenüberliegenden Seiten der Materialbahn (12) jeweils wenigstens ein einen Mikrowellenresonator umfassender Sensor (14) eingesetzt wird.

20. Verfahren nach Anspruch 19,
**dadurch gekennzeichnet,**
**dass** die auf entgegengesetzten Seiten der Materialbahn (12) vorgesehenen Sensoren (14) in Bahnlaufrichtung betrachtet zumindest soweit gegeneinander versetzt sind, dass sich deren elektromagnetische Felder nicht gegenseitig beeinflussen.

21. Verfahren nach einem der vorhergehenden Ansprüche,
**dadurch gekennzeichnet ,**
**dass** eine Anpassung der Sensorkennlinie und/oder Kalibration der Sensorwerte bezüglich der absoluten Feuchte vorgenommen wird.

22. Verfahren nach einem der vorhergehenden Ansprüche,
**dadurch gekennzeichnet,**
**dass** zur Ermittlung des in Prozent Feuchte von der Gesamtmasse angegebenen Feuchtegehalts zusätzlich das Flächengewicht der Materialbahn (12) gemessen wird.

23. Verfahren nach Anspruch 22,
**dadurch gekennzeichnet,**
**dass** die Flächengewichtsmessung in Bahnlaufrichtung betrachtet in einem Abstand von dem zur Bestimmung der Feuchte eingesetzten Sensor (14) erfolgt, in dem sich die Feuchte der Materialbahn (12) gegenüber der Feuchte der Materialbahn (12) im Bereich des zur Feuchtebestimmung eingesetzten Sensors (14) im Wesentlichen nicht ändert oder die Feuchteänderung bekannt ist.

24. Vorrichtung (10) zur Bestimmung der Feuchte einer laufenden Materialbahn (12), insbesondere Papier- oder Kartonbahn, mit einem einen Mikrowellenresonator umfassenden Sensor (14) zur Abtastung der Materialbahn (12) und einer Auswerteelektronik zur Untersuchung des Resonanzverhaltens des Mikrowellenresonators sowie zur Ermittlung der Feuchte der Materialbahn (12) anhand des Resonanzverhaltens unter Berücksichtigung des Abstandes (16) zwischen dem Mikrowellenresonator und der Materialbahn (12), insbesondere zur Durchführung des Verfahrens nach einem der vorhergehenden Ansprüche.

25. Vorrichtung nach Anspruch 24,
**dadurch gekennzeichnet,**
**dass** als Mikrowellenresonator ein Resonator in Form eines Hohlraumresonators mit Resonatorgehäuse vorgesehen ist.

26. Vorrichtung nach Anspruch 24 oder 25,
**dadurch gekennzeichnet,**
**dass** die Resonanzfrequenz des Mikrowellenresonators für einen Abstand (16) zwischen dem Mikrowellenresonator und der Materialbahn (12), wie er während der Abtastung der Materialbahn (12) gegeben ist, in einem Bereich zwischen etwa 1 und 100 GHz liegt.

27. Vorrichtung nach einem der vorhergehenden Ansprüche,
**dadurch gekennzeichnet,**
**dass** die den zur Materialbahn (12) hin offenen Mikrowellenresonator und die Materialbahn (12) umfassende Einheit abgeschlossen ist.

28. Vorrichtung nach Anspruch 27,
**dadurch gekennzeichnet,**
**dass** in einem festen Abstand (16) von der der Materialbahn (12) zugewandten offenen bzw. nicht metallisierten oder nicht dielektrischen Seite des Mikrowellenresonators ein metallisches oder dielektrisches Element (22, 26) vorgesehen ist.

29. Vorrichtung nach einem der vorhergehenden Ansprüche,
**dadurch gekennzeichnet,**
**dass** der Abstand (16) zwischen dem Mikrowellenresonator und der Materialbahn (12) zumindest im Wesentlichen konstant ist.

30. Vorrichtung nach einem der Ansprüche 24 bis 28,
**dadurch gekennzeichnet,**
**dass** auf derselben Seite der Materialbahn (12) zwei jeweils einen Mikrowellenresonator umfassende Sensoren (14, 18) vorgesehen sind, die bezüglich der Feuchte und des Abstandes unterschiedliche Empfindlichkeiten besitzen, und einer (14) der beiden Sensoren (14, 18) zur Ermittlung der Feuchte und der andere Sensor (18) zur gleichzeitigen Ermittlung des Abstandes (16) zwischen dem Mikrowellenresonator und der Materialbahn (12) vorgesehen ist.

31. Vorrichtung nach einem der vorhergehenden Ansprüche,
**dadurch gekennzeichnet,**
**dass** Mittel für eine gleichzeitige, vorzugsweise berührungslose Messung der Temperatur der Materialbahn (12) vorgesehen sind.

32. Vorrichtung nach einem der vorhergehenden Ansprüche,
**dadurch gekennzeichnet,**
**dass** die den Mikrowellenresonator und die Materialbahn (12) umfassende Einheit durch eine insbesondere metallische Stützfläche wie insbesondere den metallischen Kern einer Walze (22) abgeschlossen ist, über die die Materialbahn (12) geführt ist.

33. Vorrichtung nach Anspruch 32,
**dadurch gekennzeichnet,**
**dass** zwischen der Materialbahn (12) und der Stützfläche bzw. Walze (22) eine Bespannung (20), insbesondere ein Sieb vorgesehen ist.

34. Vorrichtung nach einem der vorhergehenden Ansprüche,
**dadurch gekennzeichnet,**
**dass** an einem freien Zug der Materialbahn (12) auf jeder den beiden einander gegenüberliegenden Seiten der Materialbahn (12) jeweils wenigstens ein einen Mikrowellenresonator umfassender Sensor (14) vorgesehen ist.

35. Vorrichtung nach Anspruch 34,
**dadurch gekennzeichnet,**
**dass** die auf entgegengesetzten Seiten der Materialbahn (12) vorgesehenen Sensoren (14) in Bahnlaufrichtung betrachtet zumindest soweit gegeneinander versetzt sind, dass sich deren elektromagnetische Felder nicht gegenseitig beeinflussen.

36. Verwendung des Verfahrens bzw. der Vorrichtung (10) nach einem der vorhergehenden Ansprüche zur Ermittlung der Feuchte eines Papierrandstreifens (28) zur Optimierung der Überführung einer Papierbahn (12) von einem Bereich einer Papiermaschine auf einen anderen.

37. Verwendung des Verfahrens bzw. der Vorrichtung (10) nach einem der vorhergehenden Ansprüche bei der Veredelung von Papier wie insbesondere dem Streichen des Papiers insbesondere zur Gelpunktbestimmung.

38. Verwendung des Verfahrens bzw. der Vorrichtung (10) nach einem der vorhergehenden Ansprüche zur Quer- und/oder Längsfeuchteprofilregelung.
